# EUROPEAN PATENT SPECIFICATION

(11) **EP 0 576 152 B1**
(45) Date of publication and mention of the grant of the patent: **06.11.2002**
(21) Application number: 93304103.0
(22) Date of filing: 26.05.1993
(51) Int. Cl.: C12N 15/57, C12N 9/64, C12Q 1/68

(54) **Protease and related DNA compounds**
Protease und verwandte DNS-Verbindungen
Protéase et des composés ADN apparentés

(30) Priority: 28.05.1992 US 891542
(43) Date of publication of application: 29.12.1993
(73) Proprietor: ELI LILLY AND COMPANY, Indianapolis, Indiana 46285 (US)
(72) Inventor: Dixon, Eric Paul, Indianapolis, Indiana 46237 (US); Johnstone, Edward Marion, Indianapolis, Indiana 46220 (US); Little, Sheila Parks, Indianapolis, Indiana 46208 (US); Norris, Franklin Harpold, Indianapolis, Indiana 46237 (US)
(74) Representative: Pritchard, Judith

(56) References cited:
- WO-A-91/13904
- WO-A-92/00374
- WO-A-92/03542
- WO-A-92/07068
- BIOCHEMICAL AND BIOPHYSICAL RESEARCH COMMUNICATIONS vol. 174, no. 2, 31 January 1991, NEW YORK, US pages 790 - 796 C.R. ABRAHAM ET AL. 'A calcium-activated protease from Alzheimer's disease brain cleaves at the N-terminus of the amyloid beta-protein'

## Description

A peptide of 42 to 43 residues known as the β-amyloid peptide (β/A4) has been implicated in Alzheimer's disease and Down's syndrome. Researchers hypothesize that abnormal accumulation of this 4 kilodalton (kd) protein in the brain is due to cleavage of a larger precursor protein, called amyloid precursor protein (APP). Normal cleavage of APP occurs within the A4 region, indicating that an alternate cleavage event occurs when the normal full length is generated. The amino terminal residue of β/A4 is most often an aspartic acid (Asp), indicating that a protease which cleaves between the methionine (Met) at position 596 [Met₅₉₆ using the numbering system according to J. Kang, et al., Nature 325:733 (1987).] and Asp₅₉₇ of APP would generate amyloid. Therefore, proteases which cleave the APP so as to generate β/A4 are important tools for characterizing Alzheimer's disease and Down's syndrome.

In the past, researchers have attempted to characterize the abnormal cleavage event through the use of classical protein purification techniques. These investigations have resulted in reports of a partially purified 68 kilodalton protease which cleaves at a Met-Asp bond of a synthetic peptide. C. Abraham, et al., Neurobiology of Aging 11A:303 (1990). In 1991, Abraham and co-workers, compared the cleavage pattern of the 68 kd protease with known serine proteases. C. Abraham, et al., Biochemical and Biophysical Research Communications, 174:790 (1991). Subsequently, the same researchers reported that the activity seen in the prior studies was actually the action of two independent proteases. One was identified as a calcium-dependent serine protease and the other a cysteine metalloprotease. C. Abraham, et al., Journal of cellular Biochemistry, 15:115 (1991); C. Abraham, et al., Journal of Neurochemistry, 57:5109 (1991). No structure or characterization of these proteases was disclosed.

WO-A-9207068 discloses the amino acid sequence of a protease which is capable fo hydrolyzing a Met-Asp bond of an amyloid-like substrate. WO-A-9113904 also discloses such a protease.

The present invention provides a new enzyme, defined in claim 1, which is structurally different from those previously described and which will cleave APP to generate amyloidogenic fragments of the size expected of a Met₅₉₆-Asp₅₉₇ cleavage. Thus, the new enzyme is very useful in furthering the characterization of Alzheimer's disease and Down's syndrome. Moreover, use of the invention may result in treatments for these or other related diseases.

To date there has been no satisfactory means of diagnosing Alzheimer's disease in a person until the dementia completely manifests itself. Confirmation of the dementia as having arisen from Alzheimer's disease requires a post-mortem examination of the brain of the afflicted patient. The instant invention provides a means of determining those patients having Alzheimer's disease or a propensity of developing Alzheimer's disease while such patients are still alive.

For purposes of clarity and as an aid in understanding the invention, as disclosed and claimed herein, the following items are defined below.

"293 cells" refers to a widely available transformed human priamry embryonal kidney cell line, as described in F.L. Graham, et al., journal of General Virology, 36:59-72 (1977). This cell line may be obtained, for example, from the American Type Culture Collection, 12301 Parklawn Drive, Rockville, Maryland, 10852-1776 (ATCC), under the accession number ATCC CRL 1573.

"AV12 cells" refers to another widely available cell line which may be obtained from the ATCC under the accession number ATCC CRL 9595.

"Amyloidogenic fragment" - An APP fragment comprising the β/A4 peptide.

"Functional compound of SEQ ID NO:1" - A compound comprising SEQ ID NO:1 which is capable of cleaving APP.

"Kunitz-like domain" - A protease inhibitor similar to soybean trypsin inhibitor or a nucleic acid sequence encoding a protease inhibitor which is similar to the soybean trypsin inhibitor. For example, the Kunitz Protease Inhibitor (KPI) region of APP as described in P. Ponte, et al., Nature 331:525 (1988), or R.E. Tanzi, et al., Nature,331:528 (1988), or N. Kitaguchi, et al., Nature, 331:530 (1988) is a Kunitz-like domain.

"pRc/Zyme" - A modified pRc/CMV eukaryotic expression vector, the pRc/CMV vector being available commerically (Invitrogen Corporation, 3985 Sorrento -Valley Blvd., Suite B, San Diego, California 92121). The plasmid pRc/Zyme comprises a human cytomegalovirus promoter and enhancer, a bovine growth hormone polyadenylation signal, a neomycin resistance gene, a beta-lactamase gene useful as an ampicillin resistance marker in E. coli, and many other features as described in the 1991 Invitrogen Catalog, page 29, as well as a NotI/SalI insert of 1451 base pairs which contains an entire Zyme coding region.

"pSZyme" - A modified E.coli cloning vector pSPORT-1™ [described in E.Y. Chen, et al., DNA, 4:165 (1985)], the plasmid pSPORT-1™ being commercially available (Gibco-BRL, 8400 Helgerman Court, Gaithersburg, Maryland 20877). This plasmid contains an origin of replication from a pUC vector, this plasmid being described in C. Yanisch-Perron, et al., Gene, 33:103-119 (1985); the beta-lactamase gene which confers ampicillin resistance; a NotI/SalI insert of 1451 base pairs which contains an entire coding region of Zyme; as well as other features.

"Part of SEQ ID NO:1" - At least 6 consecutive amino acid residues of SEQ ID NO:1.

"mRNA" - ribonucleic acid (RNA) which has been transcribed either in vivo or in vitro, including, for example, RNA transcripts prepared in vitro by transcription of coding sequences of DNA by RNA polymerase.

"SEQ ID NO:3" - The DNA sequence ATG GCT GGC GGC
ATC ATA GTC AGG G.

"SEQ ID NO:4" - The DNA sequence AAC CGA ATC TTC
AGG TCT TCC TGG GG.

"SEQ ID NO:5" - The DNA sequence TCG CTC TCT CCT
GGG GAC ACA GA.

"SEQ ID NO:6" -The DNA sequence CCA GGT GCT ATT
CCA TGT ATG TCA TAG.

"SEQ ID NO:7" -The DNA sequence TCT GTG TCC CCA
GGA GAG AGC GA.

"SEQ ID NO:8" -The DNA sequence ATA GTG AAG CTG
TCT TCT CAA T.

"Transfection" - any transfer of nucleic acid into a host cell, with or without integration of said nucleic acid into genome of said host cell.

"Zyme" - the amino acid sequence SEQ ID NO:1.

"Zyme-related band configuration" - One of two band configurations chosen from two band configurations of a herein disclosed restriction fragment polymorphism. One pattern displays a 2400 base pair band, but no 2500 base pair band. The other pattern displays a 2500 band, but no 2400 base pair band.

The present invention provides amino acid compounds which comprise the amino acid sequence 
Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys Thr
225                 230                 235                 240

Ile Gln Ala Lys.
            244
 hereinafter defined as SEQ ID NO:1.

Artisans will recognize that this protein can be synthesized by a number of different methods. All of the amino acid compounds of the invention can be made by chemical methods well known in the art, including solid phase peptide synthesis or recombinant methods. Both methods are described in U.S. Patent 4,617,149. Recombinant methods are preferred if a high yield is desired. A general method for the construction of any desired DNA sequence is provided in Brown, et al., Methods in Enzymology, 68:109 (1979).

Other routes of production are well known. Expression in eucaryotic cells can be achieved via SEQ ID NO:2, described infra. For example, the amino acid compounds can be produced in eucaryotic cells using simian virus 40, cytomegalovirus, or mouse mammary tumor virus-derived expression vectors comprising DNA which encodes SEQ ID NO:1. As is well known in the art, some viruses are also appropriate vectors. For example, the adenovirus, the vaccinia virus, the herpes virus, the baculovirus, and the rous sarcoma virus are useful. Such a method is described in U.S. Patent 4,775,624. Several alternate methods of expression are described in J. Sambrook, et al., Molecular Cloning: A Laboratory Manual, Chapters 16 and 17 (1989).

In another embodiment, the present invention encompasses nucleic acid compounds which comprise nucleic acid sequences encoding SEQ ID NO:1. As skilled artisans recognize, the amino acid compounds of the invention can be encoded by a multitude of different nucleic acid sequences due to the degeneracy of the genetic code, wherein most of the amino acids are encoded by more than one nucleic acid triplet. Because these alternate nucleic acid sequences would encode the same amino acid sequences, the present invention further comprises these alternate nucleic acid sequences. Preferably, the nucleic acid compound is DNA, sense or antisense mRNA. A most preferred embodiment of a DNA compound which encodes Zyme has this sequence: 
ATGAAGAAGC TGATGGTGGT GCTGAGTCTG ATTGCTGCAG CCTGGGCAGA 50
GGAGCAGAAT AAGTTGGTGC ATGGCGGACC CTGCGACAAG ACATCTCACC 100
CCTACCAAGC TGCCCTCTAC ACCTCGGGCC ACTTGCTCTG TGGTGGGGTC 150
CTTATCCATC CACTGTGGGT CCTCACAGCT GCCCACTGCA AAAAACCGAA 200
 
TCTTCAGGTC TTCCTGGGGA AGCATAACCT TCGGCAAAGG GAGAGTTCCC 250
AGGAGCAGAG TTCTGTTGTC CGGGCTGTGA TCCACCCTGA CTATGATGCC 300
GCCAGCCATG ACCAGGACAT CATGCTGTTG CGCCTGGCAC GCCCAGCCAA 350
ACTCTCTGAA CTCATCCAGC CCCTTCCCCT GGAGAGGGAC TGCTCAGCCA 400
ACACCACCAG CTGCCACATC CTGGGCTGGG GCAAGACAGC AGATGGTGAT 450
TTCCCTGACA CCATCCAGTG TGCATACATC CACCTGGTGT CCCGTGAGGA 500
GTGTGAGCAT GCCTACCCTG GCCAGATCAC CCAGAACATG TTGTGTGCTG 550
GGGATGAGAA GTACGGGAAG GATTCCTGCC AGGGTGATTC TGGGGGTCCG 600
CTGGTATGTG GAGACCACCT CCGAGGCCTT GTGTCATGGG GTAACATCCC 650
CTGTGGATCA AAGGAGAAGC CAGGAGTCTA CACCAACGTC TGCAGATACA 700
CGAACTGGAT CCAAAAAACC ATTCAGGCCA AG 732
 which is hereinafter defined as SEQ ID NO:2. However, also preferred are those nucleic acid compounds which are sense and antisense mRNA.

Also provided by the present invention are nucleic acid vectors comprising nucleic acids which encode SEQ ID NO:1 or a functional equivalent thereof. The preferred nucleic acid vectors are those which are DNA. Most preferred are DNA vectors which comprise the DNA sequence which is SEQ ID NO:2. An especially preferred DNA vector is the plasmid pSZyme.

E. coli/pSZyme. which contains a cloning vector comprising SEQ ID NO:2, was deposited and made part of the stock culture collection of the Northern Regional Research Laboratories (NRRL), Agricultural Research Service, U.S. Department of Agriculture, Peoria, Illinois, 61604 on April 29, 1992, under the accession number NRRL B-18971. SEQ ID NO:2 can be isolated from the plasmid, for example, as a 1451 base pair NotI/SalI restriction fragment. Other fragments are useful in obtaining SEQ ID NO:2.

Additionally, the DNA sequences can be synthesized using commercially available automated DNA synthesizers, such as the ABS (Applied Biosystems, 850 Lincoln Centre Drive, Foster City, CA 94404) 380B DNA synthesizer. The DNA sequences can also be generated by the polymerase chain reaction (PCR) as described in U.S. Patent No. 4,889,818.

Restriction fragments of these vectors are also provided. The preferred fragments are the 1451 base pair NotI/SalI restriction fragment, the 803 base pair BsrBI/Esp3I restriction fragment and the 815 base pair EcoNI/Bfal restriction fragment of pSZyme.

Moreover, DNA vectors of the present invention preferably comprise a promoter positioned to drive expression of SEQ ID NO:2, or a functional equivalent thereof. Those vectors wherein said promoter functions in human embryonic kidney cells (293 cells), AV12 cells, yeast cells, or Escherichia coli cells are preferred. The DNA expression vector most preferred is plasmid pRc/Zyme.

The plasmid pSZyme, isolatable from E. coli using standard techniques, is readily modified to construct expression vectors that produce Zyme in a variety of organisms, including, for example, E. coli, yeast of the family Saccharomycetes, and Sf9 cells derived from fall armyworm ovaries of the genus Spodoptera, (a commonly used host for baculovirus expression systems). [Commonly used references, such as Sambrook et al., supra, describe these techniques.]

The current literature contains techniques for constructing AV12 expression vectors and for transfecting AV12 host cells. See. e.g., U.S. Patent No. 4,992,373. The current literature also contains numerous techniques for constructing 293 expression vectors and for transfecting 293 host cells.

The construction protocols utilized for 293 cells can be followed to construct analogous vectors for other cell lines, merely by substituting, if necessary, the appropriate regulatory elements using well known techniques. Promoters which may be used, for example, include the thymidine kinase promoter, the metallothionin promoter, the heat shock promoter, immunoglobulin promoter, or various viral promoters such as the mouse mammary tumor virus promoter, SV40 promoter, herpesvirus promoters, or the BK virus promoters. In addition, artificially constucted promoters, derived from "consensus" sequences or created as hybrids of other promoters may be used in the course of practicing this invention.

Also disclosed herein are primers and probes, including nucleic acid compounds of at least 18 consecutive base pairs which encode SEQ ID NO:1 or a part thereof. Probes or primers which are DNA are preferred. Most preferred probes or primers are: SEQ ID NO:3 and SEQ ID NO:4. Those in the art will recognize the techniques associated with probes and primers as being well known.

For example, all or part of SEQ ID NO:3 or SEQ ID NO:4 may be used to hybridize to the coding sequence. The full length sequence can then be generated using polymerase chain reaction (PCR) amplification, using well known techniques. The full length sequence can be subsequently subcloned into any vector of choice.

Alternatively, SEQ ID NO:3 or SEQ ID NO:4 may be radioactively labeled at the 5' end in order to screen cDNA libraries by conventional means. Furthermore, any piece of Zyme-encoding DNA which has been bound to a filter may be saturated with total mRNA transcripts, in order to reverse transcribe the mRNA transcripts which bind.

Primers and probes may be obtained by means well known in the art. For example, once pSZyme is isolated, restriction enzymes and subsequent gel separation may be used to isolate the fragment of choice.

Another embodiment of the present invention is a genomic clone of Zyme. The preferred genomic clone is the 4.0 kilobase HindIII fragment from a human chromosome 19 library which hybridizes to fragments of DNA which encode SEQ ID NO:1. This can be obtained via hybridization with SEQ ID NO:2, or parts thereof. For example, SEQ ID NO:3 and SEQ ID NO:4 may be radioactively labelled and used to probe a chromosome 19 library, in order to then identify and isolate the corresponding genomic DNA.

The present invention also provides an Alzheimer's diagnostic assay as defined in claim 8. In a preferred embodiment, donor human DNA is:
1) digested with the restriction enzyme Tag I;
2) hybridized with labelled Zyme DNA to reveal a Zyme-related band configuration; and
3) compared to the similarly-digested and hybridized band configurations of those members of the donor's family who display or displayed the symptoms of Alzheimer's disease. The preferred Alzheimer's diagnostic assay utilizes a blood sample as the source of donor human DNA.

Since the genomic DNA is provided in the present invention and a Zyme-related restriction fragment length polymorphism is identified by the disclosure of this invention, the remainder of this procedure may be accomplished according to methods known in the art. For example, U.S. Patent 4,666,828, describes these procedures. [Numerous references, such as B. Lewin, Genes, at page 78 (1987), review restriction fragment length polymorphism techniques and theory.]

Host cells which harbor the nucleic acids provided by the present invention are also encompassed within this invention. A preferred host cell is an oocyte. A preferred oocyte is one which has been injected with sense mRNA or DNA compounds of the present invention. A still more preferred oocyte is one which has been injected with sense mRNA or DNA compounds of the present invention in conjunction with DNA or mRNA which encodes APP. Most preferred oocytes of the present invention are those which have been injected with sense mRNA.

Other preferred host cells are those which have been transfected with a vector which comprises SEQ ID NO:2. Preferred SEQ ID NO:2-transfected host cells include include 293, AV12, yeast and E. coli cells. Most preferred 293 and E. coli host cells are 293/pRc/Zyme, E. coli/pSZyme.

Also preferred is a host cell which has been co-transfected with a DNA vector which comprises SEQ ID NO:2 and a DNA vector which comprises the coding sequence of APP. 293 cells, AV12 cells, yeast cells and E. coli cells are especially useful co-transfected host cells.

An oocyte host cell can be constructed according to the procedure described in Lübbert, et al., Proceedings of the National Academy of Sciences (USA), 84:4332 (1987). DNA or RNA which encodes APP (both the 695 and 751 amino acid forms) may be obtained as described in Selkoe et al., Proceedings of the National Academy of Sciences (USA), 85:7341 (1988). Other host cell transfection is well known in the art. Co-transfection of cells may be accomplished using standard techniques. See , e.g., Gorman et al., Molecular and Cellular Biology, 2:1044 (1982).

Also disclosed herein is a process for constructing a host cell capable of expressing SEQ ID NO:1, said method comprising transfecting a host cell with a DNA vector that comprises a DNA sequence which encodes SEQ ID NO:1. A preferred method utilizes 293 cells as host cells. These 293 cells may be obtained from the ATCC under the accession number ATCC CRL 1573. Another preferred method utilizes AV12 cells as host cells. AV12 cells may be obtained from the ATCC under the accession number ATCC CRL 9595. Another preferred method utilizes yeast cells of the family Saccharomycetes or the bacterium E.coli as the host cells.

The preferred process utilizes an expression vector which comprises SEQ ID NO:2 in 293 cells. Especially preferred for this purpose is pRc/Zyme.

Another preferred process comprises (a) a DNA vector which comprises SEQ ID NO:2 and (b) a DNA expression vector which encodes the APP coding sequence. A most preferred process utilizes the DNA vector pRc/Zyme. Transfected host cells may be cultured under conditions well known to skilled artisans such that SEQ ID NO:1 is expressed, thus producing Zyme in the transfected host cell.

DNA homologous to a probe as defined above may be identified by combining test nucleic acid with the probe under hybridizing conditions and identifying those test nucleic acids which hybridize. The preferred probes for use in this method are SEQ ID NO:3 and SEQ ID NO:4. Hybridization techniques are well known in the art. See, e.g., Sambrook, et al., supra.

Assays utilizing the compounds provided by the present invention are also encompassed within this invention. The assays provided determine whether a substance is a ligand for Zyme, said method comprising contacting Zyme with said substance, monitoring Zyme activity by physically detectable means, and identifying those substances which interact with or affect Zyme.

Preferred assays of the present invention incorporate a cell culture assay, a high performance liquid chromotography (HPLC) assay or a synthetic competition assay.

Preferred cell culture assays utilize oocytes, AV12, E. coli, yeast or 293 cells which co-express nucleic acids which encode Zyme and APP. Those co-expressing cell culture assays which are preferred include those which utilize 293/pRc/Zyme. A preferred assay utilizes yeast cells, and a DNA compound which encodes amino acids 587 to 606 of APP. One method of performing the yeast assay is described in Smith and Kohorn, Proceedings of the National Academy of Sciences, USA, 88:5159 (1991), using Zyme-encoding DNA and APP-encoding DNA which comprises the Met₅₉₆/Asp₅₉₇ cleavage site codons.

Most preferred oocyte assays co-express mRNA. Most preferred cell culture assays utilize Western blot analysis or radiolabelled APP as the physically detectable means. A preferred HPLC assay is one wherein the substrate utilized is a full length, eukaryotically-derived APP.

The most preferred synthetic competition assay is one wherein the substance competes with the Kunitz-like domain gene product for binding to Zyme. The most preferred Zyme/Kunitz domain competition assay is one wherein APP is labelled with radioisotope.

The cell culture assays may be accomplished according to the procedures detailed by F. Ausubel, et al., Current Protocols in Molecular Biology, (1989) at pages 9.1-9.5. The HPLC assay may be performed essentially as described in Hirs and Timasheff, eds, Methods in Enzymology, Volume 91, Sections V and VI (1983). The Zyme/Kunitz-like domain binding or competition assay may be performed as described by J. Bennet and H. Yamamura, Meurotransmitter Receptor Binding, (1985) Chapter 3.

Also disclosed herein is a method for identifying or purifying Zyme, which comprises saturating test protein with anti-Zyme antibody, eliminating anti-Zyme antibody which fails to bind, and detecting the anti-Zyme antibody which remains bound. Antibody imaging techniques are known in the art.

The following are examples of aspects of the present invention. These examples are illustrative only and are not intended to limit the scope of the invention in any way.

### Example 1

### Production of Zyme in 293 cells

A lyophilized aliquot of E. coli pSZyme can be obtained from the Northern Regional Research Laboratories, Peoria, Ilinois, USA 61604, under the accession number NRRL B-18971 and used directly as the culture in the process described below. This culture has been deposited with the NRRL

Plasmid pSZyme was isolated from a culture of E. coli/pSZyme by cesium chloride purification. Plasmid pSZyme was then digested with SalI and NotI. The resulting fragment was linear. DNA ligase was used to ligate this SalI-NotI fragment and a SalI-HindIII linker into a previously linearized pRc plasmid™. (Invitrogen, catalog #V750-20)

Competent E. coli cells were then transfected with the newly created pRc/Zyme vector which contained SEQ ID NO:2 and selected for those cells which contained the ampicillin resistance gene by growing on ampicillin-containing medium.

After transfection of the pRc/Zyme vector into E. coli, a subsequent plasmid preparation was made in order to isolate the pRc/Zyme vector. In order to transfect 293 cells with the pRc/Zyme vector, the procedure developed by Chen and Okayama was employed. C. Chen and H. Okayama, Molecular and Cellular Biology, 7:2745 (1987). These cells were used in the cell culture assay as described in Example 2.

Selection on the antibiotic G418 (geneticin) was included in this step to produce stable transformants in 293 cells. The colonies which grew in the presence of G418 were then used as a source of Zyme.

### Example 2

### Cell Culture Assay

Human embryonic kidney cells (293 cells) were co-transfected with pRcZyme and an APP-encoding vector. On one occasion, a vector encoding the 695 amino acid APP (which lacks a Kunitz-like domain) was cotransfected with pRcZyme. On another occasion, a vector encoding the 751 amino acid APP (with the Kunitz-like domain) was cotransfected with pRcZyme.

Transfection was achieved using standard calcium phosphate transfection. Other transfection protocols, such as described by Sambrook, et al., supra, are also effective. Amyloidogenic fragments were detected when the 695 amino acid (without KPI) APP coding sequence was used, via Western Blot analysis, as described in Sambrook, et al., supra, using antisera to the carboxy-terminal amino acids of the APP protein. Anti BX6, as decribed in T Oltersdorf, et al., Journal of Biological Chemistry, 265:4492-4497 (1991), was used in this procedure. Amyloidogenic fragments were not detected when the 751 amino acid (with KPI) APP was used.

### Example 3

### HPLC Assay

Full length APP is produced in cells which have been infected with APP-encoding baculovirus. This procedure is accomplished according to J. Knops, et al., Journal of Biological Chemistry, 266:7285 (1991),. APP is then incubated in the presence of active Zyme and test compound. APP fragments are subsequently separated by high performance liquid chromotography. Each pooled fragment is then microsequenced using standard, such as those of Hirs and Timasheff, eds, Methods in Enzymology, Vol. 91 Sections V and VI, (1983). The quantity of amyloidogenic fragments (those which terminate at either Met₅₉₆ or Asp₅₉₇₎ generated are compared to the quantity generated in the absence of test compound to determine the ability of the test compound to affect Zyme.

### Example 4

### Zyme/Kunitz-like Domain Competition Assay

A peptide representing the KPI domain of APP is synthesized and labelled with the isotope iodine-125 (¹²⁵I). Competition binding assays are then conducted according to J.P. Bennet and H. Yamamura, Neurotransmitter Receptor Binding 61 (1985). Zyme is then bound to plastic microtitre wells as in the traditional ELISA assay. One such typical protocol for this step is described in F. Ausubel F., Current Protocols in Molecular Biology, 2:11.1-11.3 (1989). Radiolabelled KPI domain and unlabelled competitor compound is subsequently added to the wells of the 96-well microtitre plate. The wells are then washed. The remaining isotope is recorded in order to calculate the relative affinity of the unlabelled competitor compound to Zyme.

### Example 5

### Isolating the Genomic Clone

A genomic library specific for human chromosome 19 genomic library in Charon 21A bacteriophage was purchased from the American Type Culture Collection, 12301 Parklawn Drive, Rockville, Maryland, USA 20852, (ATCC) (Catalog number 57711). These phage were transfected into E. coli K802 rec A- host strain (Cat. no. 47026). The titre of the phage was 6.5-7.0 X 10⁴ plaque forming units per microliter. A genomic clone of the gene encoding Zyme was isolated by conventional screening of phage libraries (See, e.g., Sambrook et al., Molecular Cloning: A Laboratory Manual 2.6-2.114, 1989).

A radiolabelled cDNA probe was synthesized utilizing the polymerase chain reaction (such as that described by schowalter and Sommer, Analytical Biochemistry, 177:90-94, 1989) by specifically annealing SEQ ID NO: 5 and SEQ ID NO: 6 primers to an EcoRI/NotI purified (Bio-Rad Laboratories, P.O. Box 708, Rockville Centre, New York USA, 11571, catalog number 732-6010) pRc-Zyme DNA fragment.

Hybridization and washing was carried out at 65°C as described in the Zeta-Probe™ blotting membrane instruction manual (Bio-Rad, catalog number 164-0153). Putative primary Zyme bacteriophage were stored in SM buffer containing 2-3 drops of chloroform. A single homogenous plaque (711-4) was subsequently isolated from a tertiary screen. Isolation of lambda bacteriophage DNA positive by in situ hybridization to Zyme was accomplished using standard techniques.

Purified lambda phage Zyme DNA was digested with HindIII and electrophoresed on a 1% agarose/TBE (0.1 M Tris-HCl pH 8.3, 0.1 M boric acid, 1 mM ethylenediaminetetraacetic acid) gel. Separated DNA was then transferred onto a Zeta-Probe™ blotting membrane (0.5x TBE running buffer, constant 80 volts for 1 hour) as described in section 2.5 of the Zeta-Probe™ instruction manual using non-denaturing conditions, then denatured (0.4M NaOH for 10 minutes) as described in section 2.8 of the Zeta-Probe™ instruction manual.

A radiolabelled probe encompassing the BamHI/XbaI fragment of pRc/Zyme was used with a random primed DNA labelling kit (such as that which is commercially available by Boehringer Mannheim Corporation, 9115 Hague Road, P.O. Box 50414, Indianapolis, Indiana, USA 46250-0414, catalog number 1004760) to determine if the 3' coding sequence was found in our clone. Hybridization and washing to the above Zeta-Probe™ membrane was performed as previously described and autoradiography revealed homology to the 3' region of Zyme.

To confirm that phage 711-4 contained the 5' Zyme coding region, the polymerase chain reaction using SEQ ID NO:7 and SEQ ID NO:8 was again utilized to specifically amplify a 470 base pair band from tertiary plaque purified chromosome 19 Zyme phage DNA according to Kainz, et al., Analytical Biochemistry, 202:46 (1992). This DNA fragment was purified, then subcloned into the pUC 19 expression plasmid, described supra. The identity of the DNA sequences corresponding to sequences 1 to 33 of the 5' Zyme cDNA coding region and an additional 272 nucleotides upstream of the 5' Zyme coding region were confirmed by DNA sequence analysis, using standard techniques.

### Plasmid Deposits

Under the provisions of the Budapest Treaty on the International Recognition of the Deposit of Microorganisms for Purposes of Patent Procedures the following culture has been deposited with the permanent culture collection of the Northern Regional Research Center (NRRL), Agricultural Research Service, U.S. Department of Agriculture, 1815 N. University Street, Peoria, Illinois, 61604:

| Deposited Material | Accession Number |
|---|---|
| E. coli K12/ pSZyme | NRRL B-18971 |

### SEQUENCE LISTING

(1) GENERAL INFORMATION:
   (i) APPLICANT: ELI LILLY AND COMPANY
   (ii) TITLE OF INVENTION: PROTEASE AND RELATED DNA COMPOUNDS
   (iii)NUMBER OF SEQUENCES: 8
   (iv) CORRESPONDENCE ADDRESS:
      (A) ADDRESSEE: K. G. TAPPING
      (B) STREET: ERL WOOD MANOR
      (C) CITY: WINDLESHAM
      (D) STATE: SURREY
      (E) COUNTRY: UNITED KINGDOM
      (F) ZIP: GU20 6PH
   (v) COMPUTER READABLE FORM:
      (A) MEDIUM TYPE: Diskette
      (B) COMPUTER: Macintosh
      (C) OPERATING SYSTEM: Macintosh 7.0
      (D) SOFTWARE: Microsoft Word
   SEQ ID NO:1 
Met Lys Lys Leu Met Val Val Leu Ser Leu Ile Ala Ala Ala Trp Ala
                5                   10                  15
Glu Glu Gln Asn Lys Leu Val His Gly Gly Pro Cys Asp Lys Thr Ser
            20                  25                  30
His Pro Tyr Gln Ala Ala Leu Tyr Thr Ser Gly His Leu Leu Cys Gly
        35                  40                  45
Gly Val Leu Ile His Pro Leu Trp Val Leu Thr Ala Ala His Cys Lys
    50                  55                  60
Lys Pro Asn Leu Gln Val Phe Leu Gly Lys His Asn Leu Arg Gln Arg
65                  70                  75                  80
Glu Ser Ser Gln Glu Gln Ser Ser Val Val Arg Ala Val Ile His Pro
                85                  90                  95
Asp Tyr Asp Ala Ala Ser His Asp Gln Asp Ile Met Leu Leu Arg Leu
            100                 105                 110
Ala Arg Pro Ala Lys Leu Ser Glu Leu Ile Gln Pro Leu Pro Leu Glu
        115                 120                 125
Arg Asp Cys Ser Ala Asn Thr Thr Ser Cys His Ile Leu Gly Trp Gly
    130                 135                 140
Lys Thr Ala Asp Gly Asp Phe Pro Asp Thr Ile Gln Cys Ala Tyr Ile
145                 150                 155                 160
His Leu Val Ser Arg Glu Glu Cys Glu His Ala Tyr Pro Gly Gln Ile
                165                 170                 175
Thr Gln Asn Met Leu Cys Ala Gly Asp Glu Lys Tyr Gly Lys Asp Ser
            180                 185                 190
Cys Gln Gly Asp Ser Gly Gly Pro Leu Val Cys Gly Asp His Leu Arg
        195                 200                 205
Gly Leu Val Ser Trp Gly Asn Ile Pro Cys Gly Ser Lys Glu Lys Pro
    210                 215                 220
Gly Val Tyr Thr Asn Val Cys Arg Tyr Thr Asn Trp Ile Gln Lys Thr
225                 230                 235                 240
Ile Gln Ala Lys
            244
 SEQ ID NO:2 
ATGA AGAAGCTGAT 14
GGTGGTGCTG AGTCTGATTG CTGCAGCCTG GGCAGAGGAG CAGAATAAGT TGGTGCATGG 74
CGGACCCTGC GACAAGACAT CTCACCCCTA CCAAGCTGCC CTCTACACCT CGGGCCACTT 134
GCTCTGTGGT GGGGTCCTTA TCCATCCACT GTGGGTCCTC ACAGCTGCCC ACTGCAAAAA 194
ACCGAATCTT CAGGTCTTCC TGGGGAAGCA TAACCTTCGG CAAAGGGAGA GTTCCCAGGA 254
GCAGAGTTCT GTTGTCCGGG CTGTGATCCA CCCTGACTAT GATGCCGCCA GCCATGACCA 314
GGACATCATG CTGTTGCGCC TGGCACGCCC AGCCAAACTC TCTGAACTCA TCCAGCCCCT 274
TCCCCTGGAG AGGGACTGCT CAGCCAACAC CACCAGCTGC CACATCCTGG GCTGGGGCAA 334
GACAGCAGAT GGTGATTTCC CTGACACCAT CCAGTGTGCA TACATCCACC TGGTGTCCCG 394
TGAGGAGTGT GAGCATGCCT ACCCTGGCCA GATCACCCAG AACATGTTGT GTGCTGGGGA 454
TGAGAAGTAC GGGAAGGATT CCTGCCAGGG TGATTCTGGG GGTCCGCTGG TATGTGGAGA 514
CCACCTCCGA GGCCTTGTGT CATGGGGTAA CATCCCCTGT GGATCAAAGG AGAAGCCAGG 574
AGTCTACACC AACGTCTGCA GATACACGAA CTGGATCCAA AAAACCATTC AGGCCAAG 632
 SEQ ID NO:3 ATG GCT GGC GGC ATC ATA GTC AGG G 25 SEQ ID NO:4 AAC CGA ATC TTC AGG TCT TCC TGG GG 26 SEQ ID NO:5 TCG CTC TCT CCT GGG GAC ACA GA 23 SEQ ID NO:6 CCA GGT GCT ATT CCA TGT ATG TCA TAG 27 SEQ ID NO:7 TCT GTG TCC CCA GGA GAG AGC GA 23 SEQ ID NO:8

ATA GTG AAG CTG TCT TCT CAA T 22

## Claims

1. An amino acid compound functional as a protease which is capable of cleaving amyloid precursor protein (APP) to generate amyloidogenic fragments of the size expected of a Met₅₉₆-Asp₅₉₇ cleavage and which comprises the amino acid sequence 
Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys Thr
225                 230                 235                 240

Ile Gln Ala Lys
            244
 hereinafter defined as SEQ ID NO:1.

2. A nucleic acid compound which comprises a nucleic acid sequence which encodes for a compound of claim 1 which is capable of cleaving APP to generate amyloidogenic fragments of the size expected of a Met₅₉₆-Asp₅₉₇ cleavage.

3. A nucleic acid compound as claimed in Claim 2 which comprises the sequence 
ATGAAGAAGC TGATGGTGGT GCTGAGTCTG ATTGCTGCAG CCTGGGCAGA 50
GGAGCAGAAT AAGTTGGTGC ATGGCGGACC CTGCGACAAG ACATCTCACC 100
CCTACCAAGC TGCCCTCTAC ACCTCGGGCC ACTTGCTCTG TGGTGGGGTC 150
CTTATCCATC CACTGTGGGT CCTCACAGCT GCCCACTGCA AAAAACCGAA 200
TCTTCAGGTC TTCCTGGGGA AGCATAACCT TCGGCAAAGG GAGAGTTCCC 250
AGGAGCAGAG TTCTGTTGTC CGGGCTGTGA TCCACCCTGA CTATGATGCC 300
GCCAGCCATG ACCAGGACAT CATGCTGTTG CGCCTGGCAC GCCCAGCCAA 350
ACTCTCTGAA CTCATCCAGC CCCTTCCCCT GGAGAGGGAC TGCTCAGCCA 400
ACACCACCAG CTGCCACATC CTGGGCTGGG GCAAGACAGC AGATGGTGAT 450
TTCCCTGACA CCATCCAGTG TGCATACATC CACCTGGTGT CCCGTGAGGA 500
GTGTGAGCAT GCCTACCCTG GCCAGATCAC CCAGAACATG TTGTGTGCTG 550
GGGATGAGAA GTACGGGAAG GATTCCTGCC AGGGTGATTC TGGGGGTCCG 600
CTGGTATGTG GAGACCACCT CCGAGGCCTT GTGTCATGGG GTAACATCCC 650
CTGTGGATCA AAGGAGAAGC CAGGAGTCTA CACCAACGTC TGCAGATACA 700
CGAACTGGAT CCAAAAAACC ATTCAGGCCA AG 732
 hereinafter defined as SEQ ID NO:2.

4. A nucleic acid vector which comprises the nucleic acid compound of Claim 3.

5. A DNA vector of Claim 4 which is pSZyme (NRRLB-18971).

6. A host cell transfected with a nucleic acid vector of Claim 4.

7. A genomic clone encoding the amino acid compound of claim 1 which comprises a 4.0 kilobase HindIII fragment from a human chromosome 19 library which hybridizes to fragments of DNA of the compound of claim 3.

8. A process for diagnosing Alzheimer's disease or a propensity to develop Alzheimer's disease in a patient which comprises
a) securing DNA from said patient;
b) digesting said DNA with a restriction enzyme;
c) hybridizing said digested DNA with a labeled nucleotide sequence corresponding to the compound of SEQ ID NO:2, and
d) comparing pattern of hybridization to similarly-digested and hybridized band configurations of those members of the donor's family who display or have displayed the symptoms of Alzheimer's disease.

9. An assay for determining whether a test substance is a functional ligand for a protein of SEQ ID NO:1, said method comprising
a) contacting the protein of SEQ ID NO:1 with said test substance;
b) monitoring the protein's activity by physically detectable means; and
c) identifying those substances which interact with or affect the activity of the protein relative to a control which receives no test substance.

10. A method for expressing a nucleic acid sequence as claimed in Claim 2 in a transfected host cell, said method comprising culturing said transfected host cell under conditions suitable for gene expression.

## Patentansprüche

1. Aminosäureverbindung, die als Protease funktionsfähig ist, welche zur Spaltung eines Amyloidvorläuferproₜeinₛ (APP) unter Bildung von amyloidogenen Fragmenten einer Größe fähig ist, die bei einer Met₅₉₆-Asp₅₉₇ Spaltung erwartet wird und die die folgende Aminosäuresequenz umfaßt 
Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys Thr
225                 230                 235                 240

Ile Gln Ala Lys
            244
 welche hierin als SEQ ID. Nr. 1 definiert wird.

2. Nukleinsäureverbindung, die eine Nukleinsäuresequenz umfaßt, die für eine Verbindung nach Anspruch 1 kodiert, welche zur Spaltung von APP unter Bildung von amyloidogenen Fragmenten der Größe fähig ist, die bei einer Met₅₉₆-Asp₅₉₇ Spaltung erwartet wird.

3. Nukleinsäureverbindung nach Anspruch 2, die die folgende Sequenz umfaßt 
ATGAAGAAGC TGATGGTGGT GCTGAGTCTG ATTGCTGCAG CCTGGGCAGA 50
GGAGCAGAAT AAGTTGGTGC ATGGCGGACC CTGCGACAAG ACATCTCACC 100
CCTACCAAGC TGCCCTCTAC ACCTCGGGCC ACTTGCTCTG TGGTGGGGTC 150
CTTATCCATC CACTGTGGGT CCTCACAGCT GCCCACTGCA AAAAACCGAA 200
TCTTCAGGTC TTCCTGGGGA AGCATAACCT TCGGCAAAGG GAGAGTTCCC 250
AGGAGCAGAG TTCTGTTGTC CGGGCTGTGA TCCACCCTGA CTATGATGCC 300
GCCAGCCATG ACCAGGACAT CATGCTGTTG CGCCTGGCAC GCCCAGCCAA 350
ACTCTCTGAA CTCATCCAGC CCCTTCCCCT GGAGAGGGAC TGCTCAGCCA 400
ACACCACCAG CTGCCACATC CTGGGCTGGG GCAAGACAGC AGATGGTGAT 450
TTCCCTGACA CCATCCAGTG TGCATACATC CACCTGGTGT CCCGTGAGGA 500
GTGTGAGCAT GCCTACCCTG GCCAGATCAC CCAGAACATG TTGTGTGCTG 550
GGGATGAGAA GTACGGGAAG GATTCCTGCC AGGGTGATTC TGGGGGTCCG 600
CTGGTATGTG GAGACCACCT CCGAGGCCTT GTGTCATGGG GTAACATCCC 650
CTGTGGATCA AAGGAGAAGC CAGGAGTCTA CACCAACGTC TGCAGATACA 700
CGAACTGGAT CCAAAAAACC ATTCAGGCCA AG 732
 welche hierin als SEQ ID Nr. 2 definiert wird.

4. Nukleinsäurevektor, der die Nukleinsäureverbindung nach Anspruch 3 umfaßt.

5. DNA Vektor nach Anspruch 4, der pSZyme (NRRLB-18971) ist.

6. Wirtszelle, die mit einem Nukleinsäurevektor nach Anspruch 4 transfiziert ist.

7. Genomischer Klon, der die Aminosäureverbindung nach Anspruch 1 kodiert und ein 4.0 kb HindIII Fragment von einer Genbank des Humanchromosoms 19 umfaßt, das mit DNA Fragmenten der Verbindung von Anspruch 3 hybridisiert.

8. Verfahren zur Diagnose der Alzheimerschen Erkrankung oder einer Veranlagung zur Entwicklung der Alzheimerschen Erkrankung bei einem Patienten, das umfaßt
a) Beschaffung von DNA aus diesem Patienten
b) Verdau dieser DNA mit einem Restriktionsenzym
c) Hybridisierung der verdauten DNA mit einer markierten Nukleotidsequenz die der Verbindung der SEQ ID Nr. 2 entspricht, und
d) Vergleich des Hybridisierungsmusters mit ähnlich verdauten und hybridisierten Bandenkorifigurationen dieser Vertreter der Donorfamilie, die die Symptome der Alzheimerschen Erkrankung zeigen oder gezeigt haben.

9. Test zur Bestimmung, ob eine Testsubstanz ein fünktioneller Ligand für ein Protein der SEQ ID Nr. 1 ist, wobei das Verfahren umfaßt
a) Zusammenbringen des Proteins der SEQ ID Nr. 1 mit dieser Testsubstanz
b) Verfolgen der Aktivität des Proteins durch physikalisch detektierbare Methoden, und
c) Identifizierung dieser Substanzen, die mit dem Protein wechselwirken oder die Aktivität des Proteins beeinflussen, relativ zu einer Kontrolle, die keine Testsubstanz erhält.

10. Verfahren zur Expression einer Nukleinsäuresequenz nach Anspruch 2 in einer transfizierten Wirtszelle, wobei das Verfahren die Kultivierung dieser transfizierten Wirtszelle unter Bedingungen umfaßt, die zur Genexpression geeignet sind.

## Revendications

1. Composé d'acides aminés fonctionnel à titre de protéase, qui est capable de cliver la protéine précurseur amyloïde (APP) dans le but de générer des fragments amyloïdogéniques de la dimension escomptée d'un clivage Met₅₉₆-Asp₅₉₇ et qui comprend la séquence d'acides aminés 
Met Lys Lys Leu Met Val Val Leu Ser Leu Ile Ala Ala Ala Trp Ala
1               5                   10                  15

Glu Glu Gln Asn Lys Leu Val His Gly Gly Pro Cys Asp Lys Thr Ser
            20                  25                  30

His Pro Tyr Gln Ala Ala Leu Tyr Thr Ser Gly His Leu Leu Cys Gly
        35                  40                  45

Gly Val Leu Ile His Pro Leu Trp Val Leu Thr Ala Ala His Cys Lys
    50                  55                  60

Lys Pro Asn Leu Gln Val Phe Leu Gly Lys His Asn Leu Arg Gln Arg
65                  70                  75                  80

Glu Ser Ser Gln Glu Gln Ser Ser Val Val Arg Ala Val Ile His Pro
                85                  90                  95

Asp Tyr Asp Ala Ala Ser His Asp Gln Asp Ile Met Leu Leu Arg Leu
            100                 105                 110

Ala Arg Pro Ala Lys Leu Ser Glu Leu Ile Gln Pro Leu Pro Leu Glu
        115                 120                 125

Arg Asp Cys Ser Ala Asn Thr Thr Ser Cys His Ile Leu Gly Trp Gly
    130                 135                 140

Lys Thr Ala Asp Gly Asp Phe Pro Asp Thr Ile Gln Cys Ala Tyr Ile
145                 150                 155                 160

His Leu Val Ser Arg Glu Glu Cys Glu His Ala Tyr Pro Gly Gln Ile
                165                 170                 175

Thr Gln Asn Met Leu Cys Ala Gly Asp Glu Lys Tyr Gly Lys Asp Ser
            180                 185                 190

Cys Gln Gly Asp Ser Gly Gly Pro Leu Val Cys Gly Asp His Leu Arg
        195                 200                 205

Gly Leu Val Ser Trp Gly Asn Ile Pro Cys Gly Ser Lys Glu Lys Pro
    210                 215                 220

Gly Val Tyr Thr Asn Val Cys Arg Tyr Thr Asn Trp Ile Gln Lys Thr
225                 230                 235                 240

Ile Gln Ala Lys
            244
 désignée ci-après par l'expression SEQ ID NO : 1.

2. Composé d'acides nucléiques qui comprend une séquence d'acides nucléiques qui encode un composé selon la revendication 1, qui est capable de cliver l'APP dans le but de générer des fragments amyloïdogéniques de la dimension escomptée d'un clivage Met₅₉₆-Asp₅₉₇.

3. Composé d'acides nucléiques selon la revendication 2, qui comprend la séquence 
ATGAAGAAGC TGATGGTGGT GCTGAGTCTG ATTGCTGCAG CCTGGGCAGA 50
GGAGCAGAAT AAGTTGGTGC ATGGCGGACC CTGCGACAAG ACATCTCACC 100
CCTACCAAGC TGCCCTCTAC ACCTCGGGCC ACTTGCTCTG TGGTGGGGTC 150
CTTATCCATC CACTGTGGGT CCTCACAGCT GCCCACTGCA AAAAACCGAA 200
TCTTCAGGTC TTCCTGGGGA AGCATAACCT TCGGCAAAGG GAGAGTTCCC 250
AGGAGCAGAG TTCTGTTGTC CGGGCTGTGA TCCACCCTGA CTATGATGCC 300
GCCAGCCATG ACCAGGACAT CATGCTGTTG CGCCTGGCAC GCCCAGCCAA 350
ACTCTCTGAA CTCATCCAGC CCCTTCCCCT GGAGAGGGAC TGCTCAGCCA 400
ACACCACCAG CTGCCACATC CTGGGCTGGG GCAAGACAGC AGATGGTGAT 450
TTCCCTGACA CCATCCAGTG TGCATACATC CACCTGGTGT CCCGTGAGGA 500
GTGTGAGCAT GCCTACCCTG GCCAGATCAC CCAGAACATG TTGTGTGCTG 550
GGGATGAGAA GTACGGGAAG GATTCCTGCC AGGGTGATTC TGGGGGTCCG 600
CTGGTATGTG GAGACCACCT CCGAGGCCTT GTGTCATGGG GTAACATCCC 650
CTGTGGATCA AAGGAGAAGC CAGGAGTCTA CACCAACGTC TGCAGATACA 700
CGAACTGGAT CCAAAAAACC ATTCAGGCCA AG 732
 désignée ci-après par l'expression SEQ ID NO : 2.

4. Vecteur d'acides nucléiques qui comprend le composé d'acides nucléiques selon la revendication 3.

5. Vecteur d'ADN selon la revendication 4, à savoir pSZyme (NRRLB-18971).

6. Cellule hôte transfectée avec un vecteur d'acides nucléiques selon la revendication 4.

7. Clone génomique encodant le composé d'acides aminés selon la revendication 1, qui comprend un fragment HindIII de 4,0 kb issu d'une bibliothèque du chromosome humain 19, qui s'hybride à des fragments d'ADN du composé selon la revendication 3.

8. Procédé pour le diagnostic de la maladie d'Alzheimer ou d'une propension à développer la maladie d'Alzheimer chez un patient, qui comprend les étapes consistant à
a) fixer de l'ADN provenant dudit patient ;
b) mettre ledit ADN à digérer avec une enzyme de restriction ;
c) hybrider ledit ADN mis à digérer avec une séquence nucléotidique marquée correspondant au composé de SEQ ID NO: 2 ; et
d) comparer le modèle d'hybridation à des configurations de bandes soumises à une digestion et à une hybridation similaires des membres de la famille du donneur qui manifestent ou qui ont manifesté les symptômes de la maladie d'Alzheimer.

9. Dosage pour déterminer le fait de savoir si une substance d'essai représente un ligand fonctionnel pour une protéine de SEQ ID NO : 1, ledit procédé comprenant les étapes consistant à :
a) mettre la protéine de SEQ ID NO : 1 en contact avec ladite substance d'essai ;
b) surveiller l'activité de la protéine à l'aide d'un moyen détectable par voie physique ; et
c) identifier les substances qui agissent réciproquement avec la protéine ou qui affectent l'activité de la protéine par rapport à un témoin qui ne reçoit aucune substance d'essai.

10. Procédé pour exprimer une séquence d'acides nucléiques, selon la revendication 2, dans une cellule hôte transfectée, ledit procédé comprenant la mise en culture de ladite cellule hôte transfectée dans des conditions appropriées pour l'expression génique.
